# EUROPEAN PATENT SPECIFICATION

(11) **EP 4 083 179 B1**
(45) Date of publication and mention of the grant of the patent: **25.06.2025**
(21) Application number: 20907095.2
(22) Date of filing: 18.11.2020
(51) Int. Cl.: G01N 21/64, C12Q 1/6837

(54) **NUCLEIC ACID SEQUENCE MEASURING APPARATUS AND NUCLEIC ACID SEQUENCE MEASURING METHOD**
VORRICHTUNG ZUR MESSUNG VON NUKLEINSÄURESEQUENZEN UND VERFAHREN ZUR MESSUNG VON NUKLEINSÄURESEQUENZEN
APPAREIL DE MESURE DE SÉQUENCE D'ACIDE NUCLÉIQUE ET PROCÉDÉ DE MESURE DE SÉQUENCE D'ACIDE NUCLÉIQUE

(30) Priority: 23.12.2019 JP 2019231995
(43) Date of publication of application: 02.11.2022
(73) Proprietor: Yokogawa Electric Corporation, Musashino-shi Tokyo 180-8750 (JP)
(72) Inventor: MIYAUCHI Yuki, Musashino-shi, Tokyo 180-8750 (JP); TADENUMA Takashi, Musashino-shi, Tokyo 180-8750 (JP); TAGUCHI Tomoyuki, Musashino-shi, Tokyo 180-8750 (JP)
(74) Representative: Hoffmann Eitle
(86) International application number: PCT/JP2020/042910
(87) International publication number: WO 2021/131411

(56) References cited:
- JP-A- 2002 506 656
- JP-A- 2012 118 051
- JP-A- 2012 249 804
- JP-A- 2015 043 702
- US-A1- 2005 239 104
- US-A1- 2014 011 189
- US-A1- 2017 175 174
- US-A1- 2018 251 829
- US-A1- 2018 320 223
- TADENUMA TAKASHI ET AL: "Development of a Nucleic Acid Detection System for Rapid Microbial Tests", YOKOGAWA TECHNICAL REPORT, vol. 60, no. 1, 1 January 2017 (2017-01-01), pages 7 - 12, XP055928497
- GUO Z ET AL: "DIRECT FLUORESCENCE ANALYSIS OF GENETIC POLYMORPHISMS BY HYBRIDIZATION WITH OLIGONUCLEOTIDE ARRAYS ON GLASS SUPPORTS", NUCLEIC ACIDS RESEARCH, OXFORD UNIVERSITY PRESS, GB, vol. 22, no. 24, 11 December 1994 (1994-12-11), pages 5456 - 5465, XP002006248, ISSN: 0305-1048
- TADENUMA TAKASHI, TAGUCHI TOMOYUKI: "Development of a Nucleic Acid Detection System for Rapid Microbial Tests", YOKOGAWA TECHNICAL REPORT, vol. 60, no. 1, 1 January 2017 (2017-01-01), pages 7 - 12, XP055928497

## Description

### [Technical Field]

The present disclosure relates to a nucleic acid sequence measuring apparatus and to a nucleic acid sequence measuring method.

### [Background Art]

As a method for measuring a target having a specific nucleic acid sequence included in a sample, a method in which a deoxyribonucleic acid (DNA) chip (in which a detection probe having a complementary sequence of the specific nucleic acid sequence is provided on a solid-phase surface of a substrate or the like) is utilized is widely known. This method is a method of measuring a target using the property in which a target included in a sample added to a DNA chip is collected by a detection probe of the DNA chip through hybridization. In this method, it is possible to measure an amount of the target included in the sample and perform determination concerning whether the target is included in the sample.

In the following Patent Literature 1, regarding the detection probe described above, a method for measuring a target using a nucleic acid sequence measuring device (DNA chip) in which a fluorescent probe having a fluorescent molecule added thereto and a quenching probe having a quenching molecule for quenching fluorescence of the fluorescent molecule added thereto are provided is disclosed. In this method, it is possible to measure a target without adding a fluorescent molecule to the target and cleaning a DNA chip (cleaning for removing uncollected target and the like).

### [Citation List]

### [Patent Literature]

[Patent Literature 1]
   Japanese Unexamined Patent Application Publication No. 2015-43702
[Patent Literature 2]
   US 2014/0011189 A1 relates to a sensor chip that can be used to detect the presence of target nucleic acid molecules in a sample.

### [Summary of Invention]

### [Technical Problem]

Incidentally, in the method for measuring a target using the nucleic acid sequence measuring device disclosed in the foregoing Patent Literature 1, there is a problem that the measurement accuracy deteriorates due to imperfect quenching through a quenching molecule, uneven fluorescence (variation in an amount of light), or the like.

The present invention was made in view of the above circumstances, and an object of the present invention is to provide an nucleic acid sequence measuring apparatus and a nucleic acid sequence measuring method capable of improving the measurement accuracy of a target having a specific nucleic acid sequence included in a sample compared with in the related art.

### [Solution to Problem]

In order to achieve the above object, a nucleic acid sequence measuring apparatus according to an aspect of the present invention, as defined in appended idependent claim 1, is a nucleic acid sequence measuring apparatus (1) for measuring a target (TG) having a specific nucleic acid sequence included in a sample. The nucleic acid sequence measuring apparatus may include a detector(12) configured to detect fluorescence emitted from a nucleic acid sequence measuring device (DV) which emits fluorescence due to an addition of the target, and a calculator (25) configured to measure the target based on a difference between a first amount of light indicating an amount of fluorescent light emitted from a predefined measurement region (SP) of the nucleic acid sequence measuring device at a first time point before or immediately after an addition of the sample to the nucleic acid sequence measuring device and a second amount of light indicating an amount of fluorescent light emitted from the measurement region at a second time point after a predefined time has elapsed from the addition of the sample to the nucleic acid sequence measuring device, based on a detection result of the detector.

Also, in the nucleic acid sequence measuring apparatus according to an aspect of the present invention, the detector comprises an image acquirer (12a) configured to acquire an image of an image acquisition region (BK) including at least the measurement region, and the calculator is configured to perform image processing on a first image that is an image of the image acquisition region acquired at the first time point and a second image that is an image of the image acquisition region acquired at the second time point to acquire the first amount of light and the second amount of light.

Furthermore, in the nucleic acid sequence measuring apparatus according to an aspect of the present invention, the calculator is configured to perform, as the image processing, a process (S13) of acquiring, as the first amount of light, an average gradation value of pixels forming an image of the measurement region included in the first image and a process (S14) of acquiring, as the second amount of light, an average gradation value of pixels forming an image of the measurement region included in the second image.

In the nucleic acid sequence measuring apparatus according to an aspect of the present invention, the calculator is configured to extract a pixel in which a difference in gradation value for each of the pixels between the first image and the second image exceeds a predefined first threshold value, and the calculator may be configured to perform a process (S26) of acquiring, as the first amount of light, an average gradation value of the extracted pixels of the first image, and a process (S27) of acquiring, as the second amount of light, an average gradation value of the extracted pixels of the second image.

In the nucleic acid sequence measuring apparatus according to an aspect of the present invention, the calculator may be configured to acquire a standard deviation of gradation values of pixels forming an image of the measurement region included in the first image, and configured to set the first threshold value based on the standard deviation.

In the nucleic acid sequence measuring apparatus according to an aspect of the present invention, the calculator may be configured to perform, as the image processing, a process (S33) of acquiring a difference between gradation values of pixels forming an image of the measurement region included in the first image and gradation values of pixels forming an image of the measurement region included in the second image as a difference between the first amount of light and the second amount of light.

In the nucleic acid sequence measuring apparatus according to an aspect of the present invention, the calculator may be configured to extract pixels in which the difference between the first amount of light and the second amount of light exceeds a second threshold value, and configured to measure the target based on a number of the extracted pixels.

In the nucleic acid sequence measuring apparatus according to an aspect of the present invention, the calculator may be configured to acquire a standard deviation of gradation values of pixels forming an image of a region other than the measurement region included in the first image, and configured to set the second threshold value based on the standard deviation.

In the nucleic acid sequence measuring apparatus according to an aspect of the present invention, the image acquirer may be configured to acquire images of the image acquisition region at different resolutions.

A nucleic acid sequence measuring method according to an aspect of the present invention, as defined in appended independent claim 7, is a nucleic acid sequence measuring method performed using a nucleic acid sequence measuring apparatus which measures a target (TG) having a specific nucleic acid sequence included in a sample. The nucleic acid sequence measuring method may include acquiring (S15, S28, S33) a difference between a first amount of light indicating an amount of fluorescent light emitted from a predefined measurement region of a nucleic acid sequence measuring device which emits fluorescence due to an addition of the target at a first time point before or immediately after an addition of the sample to the nucleic acid sequence measuring device and a second amount of light indicating an amount of fluorescent light emitted from the measurement region at a second time point after a predefined time has elapsed from the addition of the sample to the nucleic acid sequence measuring device, and measuring (S16, S29, S36) the target based on the difference between the first amount of light and the second amount of light.

Also, the nucleic acid sequence measuring method according to an aspect of the present invention further includes acquiring an image of an image acquisition region including at least the measurement region, and performing image processing on a first image that is an image of the image acquisition region acquired at the first time point and a second image that is an image of the image acquisition region acquired at the second time point to acquire the first amount of light and the second amount of light.

Furthermore, the nucleic acid sequence measuring method according to an aspect of the present invention further includes performing, as the image processing, a process (S13) of acquiring, as the first amount of light, an average gradation value of pixels forming an image of the measurement region included in the first image and a process (S14) of acquiring, as the second amount of light, an average gradation value of pixels forming an image of the measurement region included in the second image.

The nucleic acid sequence measuring method according to an aspect of the present invention further includes extracting a pixel in which a difference in gradation value for each of the pixels between the first image and the second image exceeds a predefined first threshold value, and performing a process (S26) of acquiring, as the first amount of light, an average gradation value of the extracted pixels of the first image, and a process (S27) of acquiring, as the second amount of light, an average gradation value of the extracted pixels of the second image.

The nucleic acid sequence measuring method according to an aspect of the present invention may further include acquiring a standard deviation of gradation values of pixels forming an image of the measurement region included in the first image, and setting the first threshold value based on the standard deviation.

The nucleic acid sequence measuring method according to an aspect of the present invention may further include performing, as the image processing, a process (S33) of acquiring a difference between gradation values of pixels forming an image of the measurement region included in the first image and gradation values of pixels forming an image of the measurement region included in the second image as a difference between the first amount of light and the second amount of light.

The nucleic acid sequence measuring method according to an aspect of the present invention may further include extracting pixels in which the difference between the first amount of light and the second amount of light exceeds a second threshold value, and measuring the target based on a number of the extracted pixels.

The nucleic acid sequence measuring method according to an aspect of the present invention may further include acquiring a standard deviation of gradation values of pixels forming an image of a region other than the measurement region included in the first image, and setting the second threshold value based on the standard deviation.

The nucleic acid sequence measuring method according to an aspect of the present invention may further include acquiring images of the image acquisition region at different resolutions.

A non-transitory computer readable storage medium is a non-transitory computer readable storage medium storing a program executed by a nucleic acid sequence measuring apparatus which measures a target (TG) having a specific nucleic acid sequence included in a sample. The program may instruct the nucleic acid sequence measuring apparatus to acquire (S15, S28, S33) a difference between a first amount of light indicating an amount of fluorescent light emitted from a predefined measurement region of a nucleic acid sequence measuring device which emits fluorescence due to an addition of the target at a first time point before or immediately after an addition of the sample to the nucleic acid sequence measuring device and a second amount of light indicating an amount of fluorescent light emitted from the measurement region at a second time point after a predefined time has elapsed from the addition of the sample to the nucleic acid sequence measuring device, and measure (S16, S29, S36) the target based on the difference between the first amount of light and the second amount of light.

In the non-transitory computer readable storage medium, the program may further instruct the nucleic acid sequence measuring apparatus to acquire an image of an image acquisition region including at least the measurement region, and perform image processing on a first image that is an image of the image acquisition region acquired at the first time point and a second image that is an image of the image acquisition region acquired at the second time point to acquire the first amount of light and the second amount of light.

Further features and embodiments of the present invention will be clear from the detailed description of embodiments which will be described later with reference to the accompanying drawings.

### [Advantageous Effects of Invention]

According to the present invention, there is an effect that the measurement accuracy of a target having a specific nucleic acid sequence included in a sample can be improved compared with in the related art.

### [Brief Description of Drawings]

FIG. 1 is a perspective view schematically showing an exterior form of a nucleic acid sequence measuring device used in an embodiment of the present invention.
FIG. 2 is a diagram schematically showing a detection probe of the nucleic acid sequence measuring device used in the embodiment of the present invention.
FIG. 3 is a block diagram showing a constitution of a main part of the nucleic acid sequence measuring apparatus according to the embodiment of the present invention.
FIG. 4 is a flowchart for describing a first nucleic acid sequence measuring method.
FIG. 5A is a diagram showing an example of an image acquired through the first nucleic acid sequence measuring method.
FIG. 5B is a diagram showing an example of the image acquired through the first nucleic acid sequence measuring method.
FIG. 6 is a flowchart for describing a second nucleic acid sequence measuring method.
FIG. 7A is a diagram showing an example of an image acquired through the second nucleic acid sequence measuring method.
FIG. 7B is a diagram showing an example of the image acquired through the second nucleic acid sequence measuring method.
FIG. 8A is a diagram showing an example a variation distribution of gradation values of pixels in a spot region before and after hybridization.
FIG. 8B is a diagram showing an example of the variation distribution of the gradation values of the pixels in the spot region before and after hybridization.
FIG. 9 is a diagram showing an example of an average gradation value and a standard deviation of pixels in a spot region before and after hybridization.
FIG. 10 is a flowchart for describing a third nucleic acid sequence measuring method.
FIG. 11 is a diagram showing an example of an image acquired through the third nucleic acid sequence measuring method.
FIG. 12A is a diagram showing an example of an image acquired using a nucleic acid sequence measuring apparatus according to a modified example.
FIG. 12B is a diagram showing an example of an image acquired using a nucleic acid sequence measuring apparatus according to a modified example.
FIG. 12C is a diagram showing an example of an image acquired using a nucleic acid sequence measuring apparatus according to a modified example.
FIG. 13 is a diagram showing an example of a variation distribution of gradation values of pixels in a spot region before and after hybridization in the case of performing capturing at a high resolution.

### [Description of Embodiments]

A nucleic acid sequence measuring apparatus, a nucleic acid sequence measuring method, and a non-transitory recording medium according to an embodiment of the present invention will be described in detail below with reference to the drawings. The outline of the embodiment of the present invention will be described first, and then the details of the embodiment of the present invention will be described below.

### [Outline]

An embodiment of the present invention is intended to improve the measurement accuracy of a target having a specific nucleic acid sequence included in a sample compared with in the related art. For example, an embodiment of the present invention enables a target to be measured even if a size of the target included in a sample is very small. To be specific, the embodiment of the present invention is realizing a high measurement accuracy by eliminating an influence of weak fluorescence (offset light) emitted from the nucleic acid sequence measuring device, uneven fluorescence (variation in amount of light), or the like as far as possible when a sample is not added.

The nucleic acid sequence measuring device disclosed in Patent Literature 1 described above is a nucleic acid sequence measuring device which includes a fluorescent probe having a fluorescent molecule added thereto and a quenching probe having a quenching molecule added thereto and in which the fluorescent probe and the quenching probe are joined so that the fluorescence of the fluorescent molecule is quenched through the quenching molecule. In this nucleic acid sequence measuring device, if a target to be measured is added, hybridization breaks the joining between the fluorescent probe and the quenching probe so that fluorescence is emitted.

For this reason, if a target is not added to the nucleic acid sequence measuring device, the fluorescence of the fluorescent molecule added to the fluorescent probe is quenched through the quenching molecule added to the quenching probe. Thus, fluorescence need not be emitted from the nucleic acid sequence measuring device. However, when the quenching of fluorescence through the quenching molecule is incomplete, offset light is emitted from the nucleic acid sequence measuring device. Such offset light becomes noise and deteriorates the measurement accuracy. For example, when the amount of targets is small, the fluorescence emitted from the nucleic acid sequence measuring device is also weak. For this reason, if this weak fluorescence is hidden in the offset light, the target cannot be measured.

Also, when a target is measured using the nucleic acid sequence measuring device, before and after hybridization, a process of comparing amounts of fluorescent light emitted from the nucleic acid sequence measuring device is performed. That is to say, a process of comparing an amount of offset light emitted from the nucleic acid sequence measuring device before hybridization with an amount of fluorescent light emitted from the nucleic acid sequence measuring device after hybridization is performed.

In the related art, the above process is performed using a nucleic acid sequence measuring device in which a block (second block) to which a sample which does not include a target is added is prepared in addition to a block (first block) to which a sample which includes a target is added. That is to say, the amount of fluorescent light (offset light) emitted from the second block is compared with the amount of fluorescent light emitted from the first block (fluorescence emitted after hybridization).

Alternatively, the above process is performed using a nucleic acid sequence measuring device to which a sample which includes a target is added and a nucleic acid sequence measuring device to which a sample which does not include a target is added. That is to say, an amount of fluorescent light (offset light) emitted from the nucleic acid sequence measuring device to which a sample which does not include a target is added is compared with an amount of fluorescent light emitted from the nucleic acid sequence measuring device to which a sample which includes a target is added (fluorescence emitted after hybridization).

However, the amount of offset light varies between blocks and between nucleic acid sequence measuring devices due to manufacturing variations or the like of the nucleic acid sequence measuring device. For this reason, if the amount of offset light is relatively small, it is possible to measure a target, but if the amount of offset light is relatively large, it may not be possible to measure a target. As described above, the measurement accuracy deteriorates in accordance with a magnitude of a variation in amount of offset light between blocks and between nucleic acid sequence measuring devices.

Also, in the nucleic acid sequence measuring device disclosed in Patent Literature 1 described above, there is also a variation in the amount of fluorescent light emitted from a spot (region in which the fluorescent probe and the quenching probe in the joining state described above are fixed on a substrate). This is due to a fixed amount of the probe in the spot, an uneven reaction, and the like. Variations in amount of light in such spots also cause deterioration in measurement accuracy.

In the embodiment of the present invention, first, a difference between a first time point indicating an amount of fluorescent light emitted from a predefined measurement region of the nucleic acid sequence measuring device at a first time point before or immediately after the addition of a sample to the nucleic acid sequence measuring device and a second amount of light indicating an amount of fluorescent light emitted from a measurement region at a second time point after a predefined time has elapsed from the addition of a sample to the nucleic acid sequence measuring device is acquired. Furthermore, a target is measured based on the difference between the first amount of light and the second amount of light.

As described above, in the embodiment of the present invention, the difference between the first amount of fluorescent light emitted from a certain measurement region at the first time point and the second amount of fluorescent light emitted from the same measurement region as described above at the second time point is acquired. Furthermore, a target is measured based on the difference between the first amount of light and the second amount of light. For this reason, it is possible to improve the measurement accuracy of the target having a specific nucleic acid sequence included in a sample compared with in the related art.

### [Embodiment]

First, a nucleic acid sequence measuring device used for measuring a nucleic acid sequence will be described below. Then, a nucleic acid sequence measuring apparatus and a nucleic acid sequence measuring method which measure a nucleic acid sequence using nucleic acid sequence measuring device described above will be described in order.

### <Nucleic acid sequence measuring device>

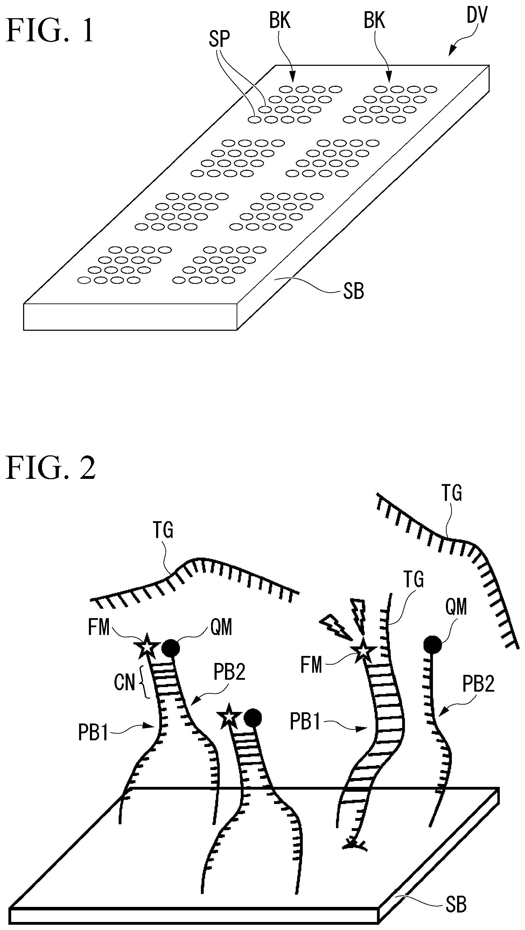

FIG. 1 is a perspective view schematically showing an exterior form of a nucleic acid sequence measuring device used in the embodiment of the present invention. As shown in FIG. 1, a nucleic acid sequence measuring device DV is, for example, a device in which a plurality of spots SP (measurement regions) are formed on a substrate SB. As the substrate SB, for example, glass, single crystals such as silicon, calcium fluoride, and sapphire, ceramics, resin materials, and the like which have plate shapes and are formed in rectangular shapes when viewed in a plan view can be used. Examples of the resin materials include cycloolefin polymers (COP) having excellent optical properties and chemical and thermal stability, cyclic olefin copolymers (COC), polycarbonate, acrylic resins, polyethylene resins, and the like. A shape of the substrate SB when viewed in a plan view may be any shape.

Each of the spots SP is a region to which a detection probe used for detecting a target to be measured is fixed. This spot SP is divided into blocks BK in units of a predefined number. A sample is added to the nucleic acid sequence measuring device DV for each of the blocks BK. Furthermore, image acquisition of the nucleic acid sequence measuring device DV is performed each of the blocks BK in many cases. That is to say, it can be said that the block BK is an image acquisition region.

FIG. 2 is a diagram schematically showing a detection probe of the nucleic acid sequence measuring device used in the embodiment of the present invention. As shown in FIG. 2, the detection probe includes a fluorescent probe PB1 and a quenching probe PB2 fixed on the substrate SB. The fluorescent probe PB1 is obtained by adding a fluorescent molecule FM to a complementary sequence of a target TG to be measured. The quenching probe PB2 is obtained by adding a quenching molecule QM to a sequence that is at least partially complementary to the complementary sequence described above of the fluorescent probe PB1.

The fluorescent probe PB1 and the quenching probe PB2 are joined so that the fluorescence of the fluorescent molecule FM is quenched through the quenching molecule QM. In the example shown in FIG. 2, the fluorescent probe PB1 and the quenching probe PB2 are joined through a joining part CN. Here, the fluorescence of the fluorescent molecule FM is quenched using the quenching molecule QM through the principle of quenching through the fluorescence resonance energy transfer.

When the target TG does not exist, the fluorescent probe PB1 and the quenching probe PB2 are joined through the joining part CN and the fluorescent molecule FM approaches the quenching molecule QM. In this state, the fluorescence of the fluorescent molecule FM is quenched using the quenching molecule QM even if the fluorescent molecule FM is irradiated with excitation light. Thus, fluorescence is not emitted.

Meanwhile, when the target TG exists, as shown in FIG. 2, the fluorescent probe PB1 having a complementary sequence of the target TG dissociates from the quenching probe PB2 and is joined to the target TG. If the target TG is joined to the fluorescent probe PB1, the joining between the fluorescent probe PB1 and the quenching probe PB2 is canceled and the fluorescent molecule FM and the quenching molecule QM are away from each other. In this state, fluorescence is emitted from the fluorescent molecule FM through irradiation with excitation light.

The complementary sequence of the target TG may be provided on the quenching probe PB2. That is to say, the quenching probe PB2 is obtained by adding the quenching molecule QM to the complementary sequence of the target TG and the fluorescent probe PB1 may be obtained by adding the fluorescent molecule FM to a sequence that is at least partially complementary to the complementary sequence described above of the quenching probe PB2.

### <Nucleic acid sequence measuring apparatus>

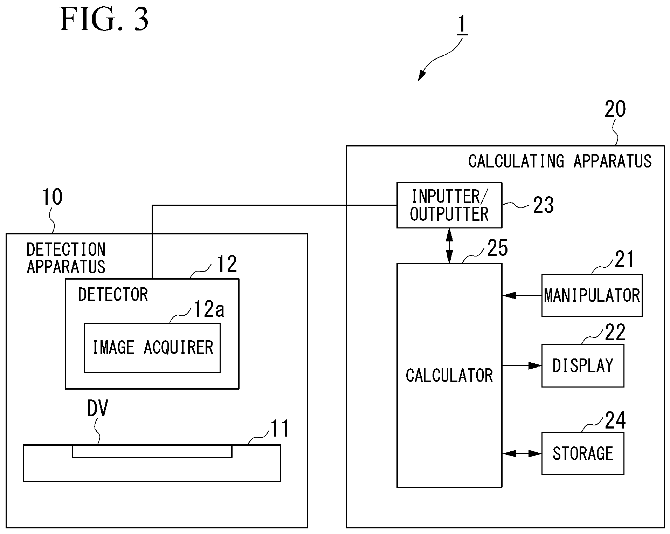

FIG. 3 is a block diagram showing a constitution of a main part of the nucleic acid sequence measuring apparatus according to the embodiment of the present invention. As shown in FIG. 3, a nucleic acid sequence measuring apparatus 1 according to the embodiment includes a detection apparatus 10 and a calculating apparatus 20 and measures a target included in a sample using the nucleic acid sequence measuring device DV described with reference to Figs. 1 and 2.

The detection apparatus 10 includes a temperature control stage 11 and a detector 12 and detects the fluorescence emitted from the nucleic acid sequence measuring device DV. The temperature control stage 11 is constituted so that the nucleic acid sequence measuring device DV can be displaced and adjusts a temperature of the displaced nucleic acid sequence measuring device DV. The temperature control stage 11 is provided to adjust the temperature of the nucleic acid sequence measuring device DV to room temperature. This is because an amount of light of the fluorescent molecule FM may change depending on the temperature in some cases. It is desirable that the temperature control stage 11 be constituted so that a sample can be agitated by vibrating or rotating the nucleic acid sequence measuring device DV to promote a hydration reaction.

The detector 12 irradiates the nucleic acid sequence measuring device DV with excitation light to detect the fluorescence emitted from the nucleic acid sequence measuring device DV. The detector 12 includes an excitation light source (not shown) and an image acquirer 12a. The excitation light source (not shown) emits excitation light with which the nucleic acid sequence measuring device DV is irradiated. For example, each of the blocks BK shown in FIG. 1 is irradiated with excitation light emitted from the excitation light source. Examples of the excitation light source (not shown) include a laser beam source configured to emit a single-wavelength laser beam or expanded light thereof, a light emitting diode (LED), a lamp configured to eject white light, a light source obtained by combining an LED and a wavelength filter, and the like.

The image acquirer 12a includes, for example, a solid-state imaging device such as a charge coupled device (CCD) or a complementary metal oxide semiconductor (CMOS) and acquires an image (two-dimensional image) of the nucleic acid sequence measuring device DV. The image acquirer 12a acquires, for example, an image for each of the blocks BK shown in FIG. 1.

The calculating apparatus 20 measures the target TG based on the detection result of the detection apparatus 10. To be specific, the calculating apparatus 20 performs a determination concerning whether the target TG is included in a sample added to the nucleic acid sequence measuring device DV and measures an amount of the target included in the sample. The calculating apparatus 20 includes a manipulator 21, a display 22, an inputter/outputter 23, a storage 24, and a calculator 25.

The manipulator 21 includes, for example, an input apparatus such as a keyboard and a pointing device and an instruction (instruction for the calculating apparatus 20) according to a manipulation of a user who uses the calculating apparatus 20 to the calculator 25. The display 22 includes, for example, a display apparatus such as a liquid crystal display apparatus and displays various information output from the calculator 25. The manipulator 21 and the display 22 may be physically separated and may be physically integrated like a touch panel type liquid crystal display apparatus having both a display function and a manipulation function.

The inputter/outputter 23 is connected to the detector 12 of the detection apparatus 10 and inputs/outputs various data to/from the detector 12. For example, the inputter/outputter 23 outputs control data for emitting excitation light from the excitation light source (not shown) to the detector 12. Furthermore, the detection apparatus 10 inputs the detection result of the detector 12 (data of an image acquired by the image acquirer 12a) to the inputter/outputter 23. After that, the inputter/outputter 23 outputs the detection result of the detector 12 to the calculator 25. The inputter/outputter 23 may be connected to the temperature control stage 11 and the calculating apparatus 20 may control a temperature of the nucleic acid sequence measuring device DV.

The storage 24 includes an auxiliary storage apparatus such as a hard disk drive (HDD) or a solid state drive (SSD) and stores various data. for example, the storage 24 stores image data output from the detector 12, various data required for the calculation of the calculator 25, data indicating the calculation result of the calculator 25, and other data. The storage 24 may store, for example, a program which realizes a function of the calculator 25.

The calculator 25 causes the image data output from the inputter/outputter 23 to be stored in the storage 24. Furthermore, the calculator 25 reads out the image data stored in the storage 24, performs image processing on the image data, and measures the target TG included in the sample added to the nucleic acid sequence measuring device DV. To be specific, the calculator 25 measures the target TG based on an amount of change in amount of fluorescent light emitted from the same spot SP before and after hybridization. In addition, the calculator 25 outputs control data for the detector 12 (for example, control data for emitting excitation light from the excitation light source (not shown)) to the inputter/outputter 23. The details of the process performed by the calculator 25 will be describe later.

A function of the calculator 25 may be realized by, for example, a central processing unit (CPU) or a micro processing unit (MPU) reading out and executing a program stored in the storage 24 in a software manner. Alternatively, the function of the calculator 25 may be realized using software of a field programmable gate array (FPGA), a large scale integration (LSI), an application specific integrated circuit (ASIC), or the like.

### <Nucleic acid sequence measuring method>

The nucleic acid sequence measuring method according to the embodiment of the present invention will be described below. In the following description, first, a measuring method (hereinafter referred to as a "first nucleic acid sequence measuring method") in which uneven fluorescence (variation in amount of light) in each of the spots SP is not taken into consideration will be described. Second, two measuring methods (hereinafter each referred to as a "second nucleic acid sequence measuring method" and a "third nucleic acid sequence measuring method") in which uneven fluorescence (variation in amount of light) in each of the spots SP is taken into consideration will be described.

### <<First nucleic acid sequence measuring method>>

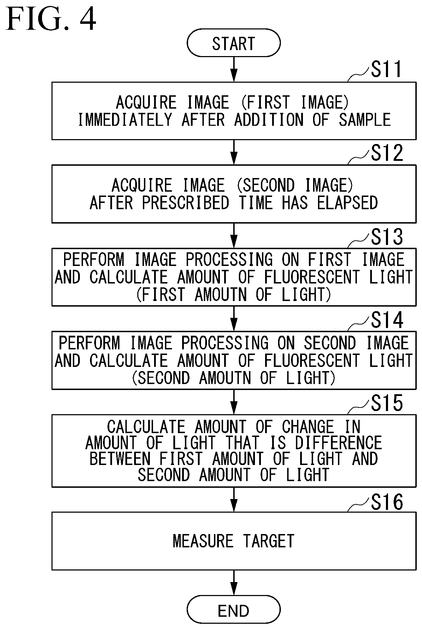

FIG. 4 is a flowchart for describing the first nucleic acid sequence measuring method. Before a process of the flowchart shown in FIG. 4 is started, the sample including the target TG is adjusted. Furthermore, work of preparing the nucleic acid sequence measuring device DV described with reference to Figs. 1 and 2 on the temperature control stage 11 of the nucleic acid sequence measuring apparatus 1 is performed. If the adjustment and work described above are completed, for example, a sample is added to one (hereinafter referred to as a "target block BK0") of a plurality of blocks BK of the nucleic acid sequence measuring device DV.

If the sample is added to the nucleic acid sequence measuring device DV, the detector 12 first acquires an image (first image) at a time point (first time point) immediately after the addition of the sample (Step S11). In this process, the control data output from the calculator 25 of the calculating apparatus 20 is input to the detector 12 via the inputter/outputter 23. Thus, the excitation light is emitted from the excitation light source (not shown) provided in the detector 12 and the target block BK0 is irradiated with the excitation light. Furthermore, the image acquirer 12a acquires an image of the target block BK0 irradiated with the excitation light.

Here, immediately after the addition of the sample means the period during which hybridization can be regarded as hardly progressing after the sample is added. It can also be said that this period is a period in which an amount of fluorescent light emitted from the nucleic acid sequence measuring device DV hardly changes even if the sample is added. This period varies depending on the properties of the nucleic acid sequence measuring device DV (the properties of the fluorescent probe PB1 and the quenching probe PB2) and an amount of the target TG included in the sample. For this reason, it is desirable that the timing for acquiring the first image be as close as possible to the time at which the sample is added.

Subsequently, the detector 12 acquires an image (second image) at a time point (second time point) after a prescribed time has elapsed from the addition of the sample (Step S12). To be specific, the image acquirer 12a acquires an image of the target block BK0 after the temperature of the nucleic acid sequence measuring device DV is raised using the temperature control stage 11 and then a time during which hybridization is considered to have sufficiently progressed has elapsed.

Here, the time during which hybridization is considered to have sufficiently progressed varies in accordance with the properties of the nucleic acid sequence measuring device DV (the properties of the fluorescent probe PB1 and the quenching probe PB2) and the amount of the target TG included in the sample. For this reason, for example, it is desirable to conduct a test in advance and set the time described above in advance.

The images acquired in Steps S11 and S12 are output from the detector 12 to the calculating apparatus 20. The detector 12 may individually output the images acquired in Steps S11 and S12 to the calculating apparatus 20 each time each step is completed. Alternatively, the detector 12 may temporarily store the image acquired in Step S11 and output the image to the calculating apparatus 20 together with the image acquired in Step S12 after the end of Step S12. The image output from the detector 12 to the calculating apparatus 20 is stored in the storage 24 of the calculating apparatus 20.

Subsequently, the calculator 25 of the calculating apparatus 20 reads out the first image stored in the storage 24, performs image processing on the first image, and calculates an amount of fluorescent light (first amount of light) emitted from each of the spots SP (Step S13). To be specific, the calculator 25 performs image processing on the image acquired in Step S11 (the image of the target block BK0) and extracts a spot region (a region presumed to be an image of the spot SP). Furthermore, the calculator 25 acquires, as the first amount of light, an average gradation value for each of the pixels forming the image of the spot SP.

Subsequently, the calculator 25 of the calculating apparatus 20 reads out the second image stored in the storage 24, performs image processing on the second image, and calculates an amount of fluorescent light (second amount of light) emitted from each of the spots SP (Step S14). To be specific, the calculator 25 performs image processing on the image acquired in Step S12 (the image of the target block BK0) and extracts a spot region. Furthermore, the calculator 25 acquires, as the second amount of light, an average gradation value for each of the pixels forming the image of the spot SP.

Also, the calculator 25 calculates an amount of change in amount of light that is a difference (amount of change in amount of light) between the average gradation value (first amount of light) calculated in Step S13 and the average gradation value (second amount of light) calculated in Step S14 (Step S15). That is to say, the calculator 25 calculates an amount of change in amount of fluorescent light emitted from the same spot SP before and after hybridization.

If the process described above is completed, the calculator 25 of the calculating apparatus 20 measures the target TG included in the sample (Step S16). To be specific, the calculator 25 determines whether the target TG is included in the sample added to the nucleic acid sequence measuring device DV. Furthermore, the calculator 25 measures an amount of the target included in the sample. In this way, the target TG included in the sample is measured.

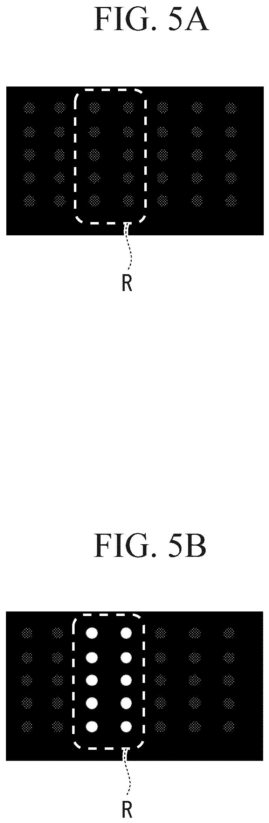

FIG. 5 is a diagram showing an example of an image acquired through the first nucleic acid sequence measuring method. FIG. 5A is, for example, an image (first image) of the target block BK0 before hybridization and FIG. 5B is, for example, an image (second image) of the target block BK0 after hybridization.

In Figs. 5A and 5B, circular regions arranged in two dimensions show an image (spot region) of the spot SP provided in the target block BK0. The color of this spot region has a gradation value (closer to black) which decreases as the amount of fluorescent light emitted from the spot SP decreases and a gradation value (closer to white) which increases as the amount of amount of fluorescent light emitted from the spot SP increases.

As shown in FIG. 5A, before hybridization, each color of the spot region is gray. This means that offset light is emitted from each of the spots SP due to the incompleteness of quenching using the quenching molecule QM provided on the quenching probe PB2. As shown in FIG. 5B, after hybridization, a color of a spot region in a region R surrounded by the broken line is white. This means that, at the spot SP in the region R, the target TG is collected using the detection probe and emits strong fluorescence.

In the process of Step S13 described above, the calculator 25 calculates, as the first amount of light, an average gradation value for each of the spot regions shown in FIG. 5A. In the process of Step S14 described above, the calculator 25 calculates, as the second amount of light, an average gradation value for each of the spot regions shown in FIG. 5B. Furthermore, in the process of Step S15 described above, the calculator 25 calculates an amount of change in amount of light that is a difference between the first amount of light and the second amount of light for each of the spot regions.

Although a total of 10 spot regions arranged in two vertical rows and five horizontal rows exist in the region R in the example shown in Figs. 5A and 5B, an amount of change in amount of light in these ten spot regions is larger than an amount of change in amount of light in the other spot regions. For this reason, in the process of Step S16 described above, the calculator 25 measures the target TG included in the sample using the amount of change in amount of light in the ten spot regions in the region R.

As described above, in the first nucleic acid sequence measuring method, the difference between the first amount of light emitted from a certain spot SP immediately after the addition of the sample (first time point) and the second amount of fluorescent light emitted from the same spot SP at a prescribed time after the addition of the sample (second time point) is acquired. Furthermore, the target is measured based on the difference between the first amount of light and the second amount of light. Thus, since an influence of offset light can be effectively eliminated, it is possible to improve the measurement accuracy of the target having a specific nucleic acid sequence included in the sample compared with in the related art.

### <<Second nucleic acid sequence measuring method>>

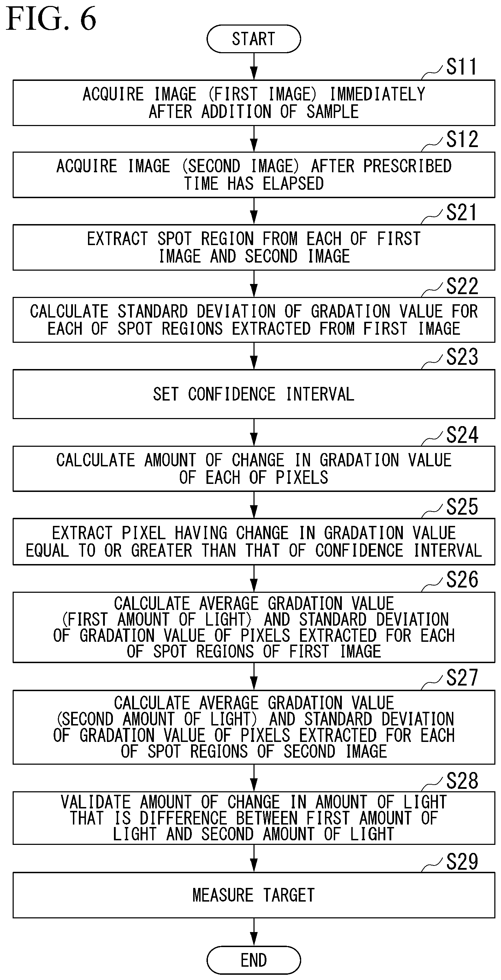

FIG. 6 is a flowchart for describing the second nucleic acid sequence measuring method. Steps of FIG. 6 that are the same as those shown in FIG. 4 will be denoted by the same reference numerals. Also in the measuring method, the sample including the target TG is adjusted before the process of the flowchart shown in FIG. 6 is started, and then the sample is added to the target block BK0.

If the sample is added to the nucleic acid sequence measuring device DV, the detector 12 first acquires an image (first image) at a time point (first time point) immediately after the addition of the sample (Step S11). Subsequently, the detector 12 acquires an image (second image) at a time point (second time point) after the sample is added and then a prescribed time has elapsed (Step S12). Since the processes of Steps S11 and S12 are the same as the processes described with reference to FIG. 4, detailed description thereof will be omitted.

Subsequently, the calculator 25 performs image processing on the first image and the second image and extracts a spot region from each of the first image and the second image (Step S21). Subsequently, the calculator 25 calculates a standard deviation of a gradation value for each of the spot regions extracted from the first image (Step S22). To be specific, the calculator 25 calculates a standard deviation of a gradation value of the pixels forming the image of the spot SP for each of the spot regions extracted from the first image. Furthermore, the calculator 25 sets a confidence interval (first threshold value) of each of the spot regions using the standard deviation of the gradation value calculated in Step S22 (Step S23).

Here, the process of Step S22 is performed to determine an amount of uneven fluorescence (variation in amount of light) occurring in each of the spot regions. The process of Step S23 is performed to extract a pixel having a certain amount of change in amount of light before and after hybridization (pixel having a large difference in gradation value) for each of the spot regions in the process of Step S25 which will be described later. Although a method for setting a confidence interval is arbitrary, for example, a confidence interval having a confidence coefficient of 0.95 can be set.

Subsequently, the calculator 25 calculates an amount of change in gradation value for each of the pixels forming the image of the spot SP for each of the spot regions extracted in the process of Step S21 (Step S24). Furthermore, the calculator 25 extracts a pixel having a change in gradation value equal to or greater than that of the confidence interval set in Step S23 for each of the spot regions extracted in the process of Step S21 (Step S25).

If the process described above is completed, the calculator 25 calculates an average gradation value (first amount of light) and a standard deviation of a gradation value of the pixels extracted in the process of Step S25 for each of the spot regions extracted from the first image in the process of Step S21 (Step S26). Similarly, the calculator 25 calculates an average gradation value (second amount of light) and a standard deviation of a gradation value of the pixels extracted in the process of Step S25 for each of the spot regions extracted from the second image in the process of Step S21 (Step S27).

Subsequently, the calculator 25 validates a difference (amount of change in amount of light) between the average gradation value (first amount of light) calculated in Step S26 and the average gradation value (second amount of light) calculated in Step S27 (Step S28). To be specific, the calculator 25 validates the difference (amount of change in amount of light) between the average gradation values described above using the standard deviations of the gradation value calculated in Steps S26 and S27. Finally, the calculator 25 measures the target TG included in the sample based on the validation result of Step S28 (Step S29). Thus, the target TG included in the sample is measured.

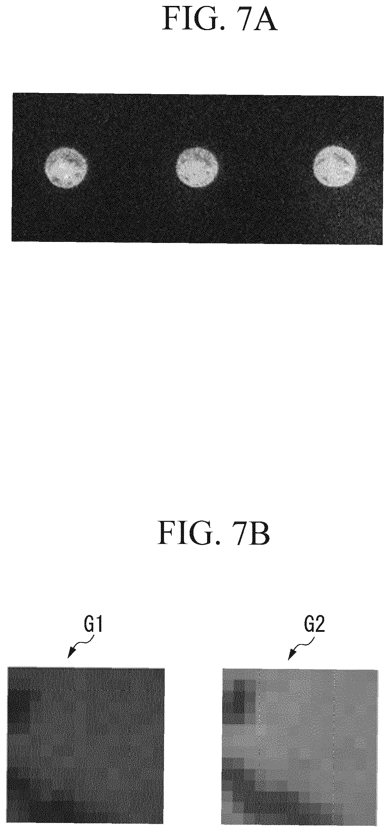

FIG. 7 is a diagram showing an example of an image acquired through the second nucleic acid sequence measuring method. FIG. 7A is, for example, a part of an image (first image) of the target block BK0 acquired before hybridization. FIG. 7B is an enlarged view of one spot regions of the images (first image, second image) acquired before and after hybridization. In FIG. 7B, an image G1 is an image before hybridization and an image G2 is an image after hybridization.

Referring to FIG. 7A, three spot regions are shown. Inside these three spot regions, the gradation values are not the same but are mottled. Furthermore, the mottled patterns in these three spot regions are not the same but different from each other. Thus, it can be seen that an amount of offset light varies not only within the spot region but also between the spot regions.

Referring to FIG. 7B, it can be seen that the image G2 is generally whitish (has a larger gradation value) than the image G1. This means that the overall amount of light is higher after hybridization than before hybridization. Furthermore, referring to FIG. 7B, the gradation values of both of the images G1 and G2 are not the same but are mottled. Thus, it can be seen that a variation in amount of fluorescent light in the spot region occurs not only before hybridization but also after hybridization.

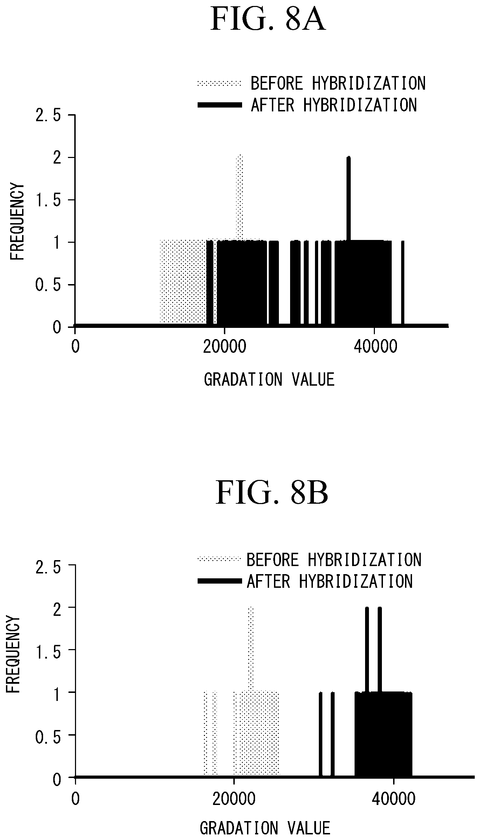

FIG. 8 is a diagram showing an example of a variation distribution of gradation values of pixels in a spot region before and after hybridization. FIG. 8A is a histogram showing variation distributions of gradation values of the images G1 and G2 shown in FIG. 7B. Meanwhile, FIG. 8B is a histogram showing variation distributions of gradation values of pixels (pixels having a large amount of gradation change) extracted from the images G1 and G2 shown in FIG. 7B. That is to say, FIG. 8B is a graph of the histogram showing the variation distributions of the gradation values for the pixels which has been subjected to the process of Step S25 shown in FIG. 6.

Referring to FIG. 8, it can be seen that, before hybridization, a distribution of the gradation values of the pixels is around "20000", and after hybridization, a distribution of the gradation values of the pixels is around "40000." Furthermore, comparing FIG. 8A with FIG. 8B, it can be seen that the variation of FIG. 8B is smaller than that of FIG. 8A and the average gradation value of FIG. 8B is larger than that of FIG. 8A.

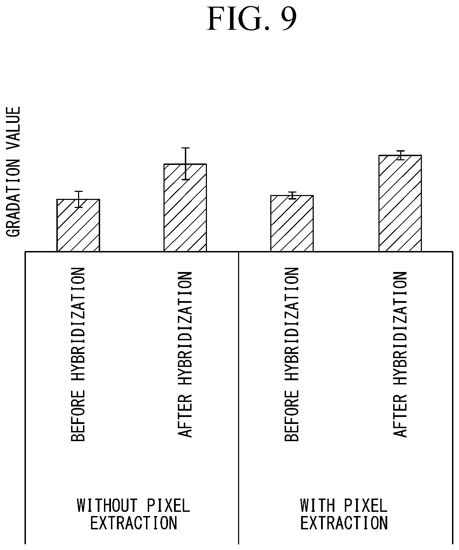

FIG. 9 is a diagram showing an example of an average gradation value and a standard deviation of pixels in a spot region before and after hybridization. In FIG. 9, the expression "without pixel extraction" is additionally provided to the average gradation value and the standard deviation of the images G1 and G2 shown in FIG. 7B. On the other hand, in FIG. 9, the expression "with pixel extraction" is additionally provided to the average gradation value and the standard deviation of the pixels (pixels having a large amount of gradation change) extracted from the images G1 and G2 shown in FIG. 7B. In FIG. 9, the average gradation value is shown by a bar graph and the standard deviation is shown by an error bar.

Referring to FIG. 9, it can be seen that, whether before or after hybridization, the average gradation value of the pixels to which the expression "with pixel extraction" is additionally provided is larger than the average gradation value of the pixels to which the expression "without pixel extraction." Furthermore, it can be seen that, whether before or after hybridization, the standard deviation of the pixels to which the expression "with pixel extraction" is additionally provided is reduced to about one-third of the standard deviation of the pixels to which the expression "without pixel extraction."

In the process of Step S28 shown in FIG. 6, the calculator 25 validates an amount of change in amount of light that is a difference between the average gradation value before hybridization and the average gradation value after hybridization using the standard deviation before and after hybridization. When the process of Step S25 shown in FIG. 6 is performed, the standard deviation can be reduced to about one-third. Thus, it is possible to improve the measurement accuracy of the target.

As described above, in the second nucleic acid sequence measuring method, first, the confidence interval is set from the standard deviation of the gradation value of the spot region extracted from the first image and the pixel having a change in gradation value equal to or greater than that of the confidence interval is extracted. Subsequently, the average gradation value (first amount of light) of the extracted pixel is acquired for each of the spot regions of the first image, the average gradation value (second amount of light) of the extracted pixel is acquired for each of the spot regions of the first image, and an amount of change in amount of light that is the difference between them is validated. Furthermore, the target is measured based on the validation result. Thus, since an influence of an amount of uneven light in the spot region can be effectively eliminated, it is possible to improve the measurement accuracy of the target having a specific nucleic acid sequence included in the sample compared with in the related art.

### <<Third nucleic acid sequence measuring method>>

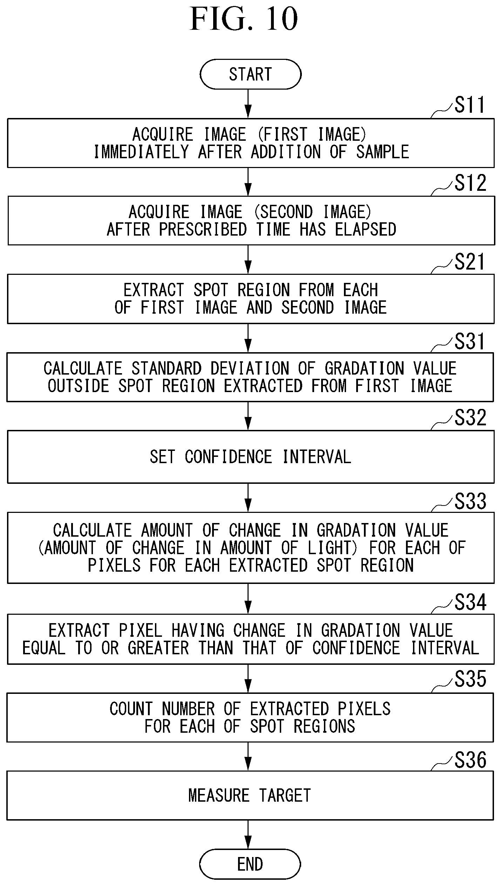

FIG. 10 is a flowchart for describing the third nucleic acid sequence measuring method. Steps of FIG. 10 that are the same as those shown in Figs. 4 and 6 will be denoted by the same reference numerals. Also in the measuring method, the same including the target TG is adjusted before the process of the flowchart shown in FIG. 10 is started, and then the sample is added to the target block BK0.

If the sample is added to the nucleic acid sequence measuring device DV, the detector 12 first acquires an image (first image) at a time point (first time point) immediately after the addition of the sample (Step S11). Subsequently, the detector 12 acquires an image (second image) at a time point (second time point) after the sample is added and then a prescribed time has elapsed (Step S12). Subsequently, the calculator 25 performs image processing on the first image and the second image and extracts a spot region from each of the first image and the second image (Step S21).

Subsequently, the calculator 25 calculates a standard deviation of a gradation value outside the spot region extracted from the first image (Step S31). To be specific, the calculator 25 calculates a standard deviation of gradation values of the pixels forming an image in an arbitrary region other than the spot region extracted from the first image. Furthermore, the calculator 25 sets a confidence interval (second threshold value) using the standard deviation of the gradation value calculated in Step S31 (Step S32).

Here, the process in Step S31 is performed, for example, to acquire an amount of variation occurring due to noise caused by the characteristics of the nucleic acid sequence measuring device DV and noise caused by the solid-state imaging device provided in the image acquirer 12a. The process of Step S32 is performed to extract a pixel in which an amount of change in amount of light before and after hybridization is large to some extent (pixel in which a difference in gradation value is large to some extent) for each of the spot regions in the process of Step S34 which will be described later. Although a method for setting a confidence interval is arbitrary, for example, a confidence interval having a confidence coefficient of 0.95 can be set.

Subsequently, the calculator 25 calculates an amount of change in gradation value for each of the pixels forming the image of the spot SP for each of the spot regions extracted in the process of Step S21 (Step S33). Furthermore, the calculator 25 extracts a pixel having a change in gradation value equal to or greater than that of the confidence interval set in Step S32 for each of the spot regions extracted in the process of Step S21 (Step S34).

If the process described above is completed, the calculator 25 counts the number of extracted pixels for each of the spot regions extracted in the process of Step S21 (Step S35). Finally, the calculator 25 measures the target TG included in the sample based on the counting result of Step S35 (Step S36). In this way, the target TG included in the sample is measured.

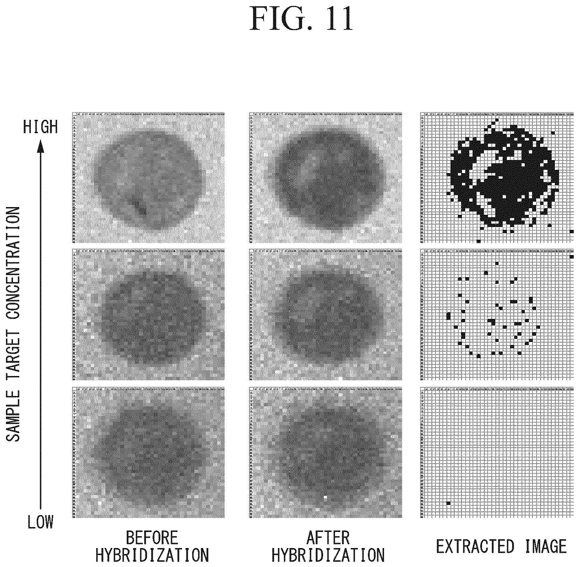

FIG. 11 is a diagram showing an example of an image acquired through the third nucleic acid sequence measuring method. FIG. 3 shows images of spots before and after hybridization acquired when three samples having different target TG concentrations are added to the nucleic acid sequence measuring device DV. First, when the concentration of the target TG is low, a change in gradation value of the image is slight before and after hybridization. For this reason, there are no pixels extracted through the process of Step S34 of FIG. 10.

Subsequently, as the concentration of the target TG increases, the number of pixels showing a large change in gradation value increases before and after hybridization. For this reason, the pixels extracted in the process of Step S34 of FIG. 10 increases as the concentration of the target TG increases. In this way, when the number of extracted pixels is counted, it is possible to measure the target TG included in the sample.

As described above, in the third nucleic acid sequence measuring method, first, the confidence interval is set from the standard deviation of the gradation value outside the spot region extracted from the first image. Subsequently, an amount of change in gradation value of each pixel is calculated for each of the extracted spot regions and a pixel having a change in gradation value equal to greater than that of the confidence interval is extracted. Furthermore, the number of extracted pixels is counted for each of the extracted spot regions and the target is measured based on the counting result. Thus, it is possible to effectively eliminate an influence of an uneven amount of light in the spot region. Therefore, it is possible to improve the measurement accuracy of the target having a specific nucleic acid sequence included in the sample compared with in the related art.

### <Modified example>

The nucleic acid sequence measuring apparatus 1 according to the embodiment of the present invention may be able to acquire images of the nucleic acid sequence measuring device DV (images of the block BK or the spot SP) at different resolutions. For example, an optical system in which an optical magnification can be changed may be constituted to be provided between the image acquirer 12a and the temperature control stage 11 (the nucleic acid sequence measuring device DV) shown in FIG. 3. Alternatively, a plurality of solid-state imaging devices having different numbers of pixels may be constituted to be provided in the image acquirer 12a shown in FIG. 3 and the solid-state imaging device may be constituted to be switchable in accordance with the resolution. This is performed to further improve the measurement accuracy of the target by photographing the spot SP at a high resolution.

For example, it is assumed that a diameter of the spot SP is 100 [µm] and a size of on pixel of the solid-state imaging device is 10 [µm] square (10×10 [µm]). When the solid-state imaging device captures an image of the spot SP at the same magnification, the number of pixels configured to capture the image of the spot SP is only about 78.5 pixels(5×5×π pixels).

Meanwhile, for example, assuming that the image of spot SP is magnified 10 times by the optical system described above, the range photographed through one pixel is assumed to be 1 [µm] square (1×1 [µm]). Thus, the number of pixels configured to capture the spot SP is about 7850 pixels (50×50×π pixels) and the resolution of the square of the magnification is obtained. When an image of the nucleic acid sequence measuring device DV can be acquired at a high resolution other than lower resolution (the same magnification) in this way, it becomes possible to more accurately capture the variation of the gradation value in the spot region.

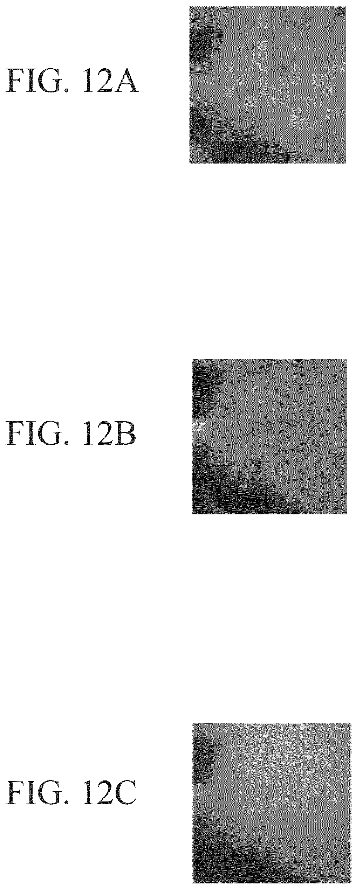

FIG. 12 is a diagram showing an example of an image acquired using a nucleic acid sequence measuring apparatus according to a modified example. FIG. 12A is an image acquired when a certain spot SP is photographed at the same magnification. FIG. 12B is an image acquired when the same spot SP as FIG. 12A is photographed at a magnification of 10 times and FIG. 12C is an image acquired when the same spot SP as FIG. 12A is photographed at a magnification of 40 times.

Referring to FIG. 12A, it can be seen that an image with a coarse mosaic can be obtained when the image is photographed at the same magnification. Referring to FIG. 12B, it can be seen that, when the image is photographed at a magnification of 10 times, although the roughness of the eyes is smaller than that of the image shown in FIG. 12A, an image which looks like a mosaic can be obtained. Meanwhile, referring to FIG. 12C, it can be seen that an image in which the gradation value changes smoothly can be obtained.

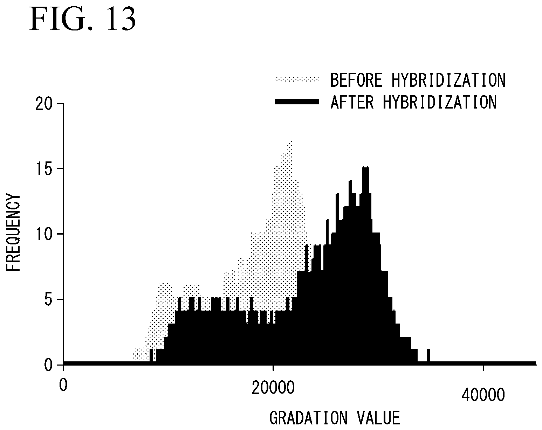

FIG. 13 is a diagram showing an example of a variation distribution of a gradation value of pixels in a spot region before and after hybridization when an image is photographed at a high resolution. Comparing FIG. 13 with FIG. 8A, it can be seen that the variation distribution of the gradation value can be accurately obtained when the image is photographed at a high resolution.

Although the nucleic acid sequence measuring apparatus and the nucleic acid sequence measuring method according to the embodiment of the present invention have been described above, the present invention is not limited to the embodiment described above and can be freely changed within the scope of the present invention, which is defined by the appended claims. For example, the image acquirer 12a described in the embodiment described above may acquire a monochrome two-dimensional image or may acquire a color two-dimensional image.

Also, the image acquirer 12a may include a highly sensitive electron multiplying CCD (EMCCD) or a digital CMOS in addition to a CCD and a customer online management system (COMS). Furthermore, the detector 12 may include a photodiode disposed one-to-one with the spot SP instead of the image acquirer 12a (or together with the image acquirer 12a).

In addition, in the embodiment described above, an example in which an image immediately after adding a sample (an image of the target block BK0) is acquired a first image has been described. However, an image before the sample is added (an image of the target block BK0) may be acquired as the first image. That is to say, the first image may be acquired at a first time point before or immediately after the addition of the sample. Here, when the image before the sample is added is acquired as the first image, it is necessary to obtain in advance a difference between an amount of light obtained before the sample is added and an amount of light obtained after the sample which does not include a target is added and provide the difference to the amount of light in the first image. Furthermore, before a sample including a target is added, an image to which a sample which does not include a target is added (an image of the target block BK0) may be acquired as the first image.

Also, in the first to third nucleic acid sequence measuring methods described above, in order to simplify the explanation, an example in which the first image and the second image are first collectively acquired and then the image processing of the first image and the second image is performed has been described. However, the image processing of the first image or the like may be performed between the acquisition of the first image and the acquisition of the second image.

Also, the method for setting a confidence interval in the third nucleic acid sequence measuring method described above may be used in the second nucleic acid sequence measuring method described above. On the other hand, the method for setting a confidence interval in the second nucleic acid sequence measuring method described above may be used in the third nucleic acid sequence measuring method described above. To be specific, the processes of Steps S22 and S23 in FIG. 6 may be replaced with the processes of Steps S31 and S32 of FIG. 10 and the processes of Steps S31 and S32 of FIG. 10 may be replaced with the processes of Steps S22 and S23 of FIG. 6.

Furthermore, the nucleic acid sequence measuring apparatus and the nucleic acid sequence measuring method according to the embodiment of the present invention can be used for dry image measurement in fluorescent molecule light amount measurement, in-liquid observation of fluorescent molecule light amount of a biochip, a real-time observation in continuous reaction, and the like. To be specific, the nucleic acid sequence measuring apparatus and the nucleic acid sequence measuring method according to the embodiment of the present invention are used, for example, for bacterial species determination through gene/polymer analysis, oncogene, animal and plant determination, and the like.

In addition, the nucleic acid sequence measuring apparatus and the nucleic acid sequence measuring method according to the embodiment of the present invention are also applied to the following solid phase methods such as a labeled antibody method used for clinical examinations and the like. For example, a fluorescence in situ hybridization (FISH) method in which the expression of a specific chromosome or gene in a tissue or cell is measured through fluorescence using a fluorescent substance. In addition to this, application to various methods such as the following methods can be mentioned as an example. That is to say, a fluorescence immunofluorescence measurement (FIA) method for measuring an antigen-antibody reaction using a fluorescent luminescent substance such as europium as a label or an indirect fluorescent antibody (IFA) method for measuring a serum (antibody) reaction in which a fluorescent substance is labeled on a pathogen serving as an antigen can be mentioned.

In this specification, terms indicating directions such as a "forward direction, a rearward direction, an upward direction, a downward direction, a rightward direction, a leftward direction, a vertical direction, a horizontal direction, a length direction, a breadth direction, a row direction, and a column direction" refer to these directions in the apparatus of the present invention. Therefore, these terms in this specification of the present invention need to be interpreted relative to each other in the apparatus of the present invention.

The term "constituted" is used to describe the constitution, the elements, or a part of the apparatus configured to perform the functions of the present invention.

Moreover, the word expressed as a "means plus function" in the claim need to include any structure which can be used to perform the functions included in the present invention.

The term "unit" is used to refer to a constituent element, a unit, hardware, or a part of software programmed to perform a desired function. Typical examples of hardware are devices and circuits, but not limited to these.

Although preferable examples of the present invention have been described above, the present invention is not limited to these examples. Additions, omissions, replacements, and other changes of a constitution are possible without departing from the scope of the present invention as defined by the appended claims. The present invention is not limited by the above description, but only the scope of the appended claims.

### [Reference Signs List]

1 Nucleic acid sequence measuring apparatus
12 Detector
12a Image acquirer
25 Calculator
BK Block
DV Nucleic acid sequence measuring device
SP Spot
TG Target

## Claims

1. A nucleic acid sequence measuring apparatus (1) for measuring a target (TG) having a specific nucleic acid sequence included in a sample, the nucleic acid sequence measuring apparatus (1) comprising:
a detector (12) configured to detect fluorescence emitted from a nucleic acid sequence measuring device (DV) which emits fluorescence due to an addition of the target (TG); and
a calculator (25) configured to measure the target (TG) based on a difference between a first amount of light indicating an amount of fluorescent light emitted from a predefined measurement region (SP) of the nucleic acid sequence measuring device (DV) at a first time point before or immediately after an addition of the sample to the nucleic acid sequence measuring device (DV) and a second amount of light indicating an amount of fluorescent light emitted from the measurement region (SP) at a second time point after a predefined time has elapsed from the addition of the sample to the nucleic acid sequence measuring device (DV), based on a detection result of the detector (12),
wherein the detector (12) comprises an image acquirer (12a) configured to acquire an image of an image acquisition region (BK) including at least the measurement region (SP), and
wherein the calculator (25) is configured to perform image processing on a first image that is an image of the image acquisition region (BK) acquired at the first time point and a second image that is an image of the image acquisition region (BK) acquired at the second time point to acquire the first amount of light and the second amount of light,
**characterized in that**
the calculator (25) is configured to perform, as the image processing, a process of acquiring, as the first amount of light, an average gradation value of pixels forming an image of the measurement region (SP) included in the first image and a process of acquiring, as the second amount of light, an average gradation value of pixels forming an image of the measurement region (SP) included in the second image,
**in that** the calculator (25) is configured to extract a pixel in which a difference in gradation value for each of the pixels between the first image and the second image exceeds a predefined first threshold value, and
**in that** the calculator (25) is configured to perform a process of acquiring, as the first amount of light, an average gradation value of the extracted pixels of the first image, and a process of acquiring, as the second amount of light, an average gradation value of the extracted pixels of the second image.

2. The nucleic acid sequence measuring apparatus (1) according to claim 1,
wherein the calculator (25) is configured to acquire a standard deviation of gradation values of pixels forming an image of the measurement region (SP) included in the first image, and configured to set the first threshold value based on the standard deviation.

3. The nucleic acid sequence measuring apparatus (1) according to claim 1,
wherein the calculator (25) is configured to perform, as the image processing, a process of acquiring a difference between gradation values of pixels forming an image of the measurement region (SP) included in the first image and gradation values of pixels forming an image of the measurement region (SP) included in the second image as a difference between the first amount of light and the second amount of light.

4. The nucleic acid sequence measuring apparatus (1) according to claim 3,
wherein the calculator (25) is configured to extract pixels in which the difference between the first amount of light and the second amount of light exceeds a second threshold value, and configured to measure the target (TG) based on a number of the extracted pixels.

5. The nucleic acid sequence measuring apparatus (1) according to claim 4,
wherein the calculator (25) is configured to acquire a standard deviation of gradation values of pixels forming an image of a region other than the measurement region (SP) included in the first image, and configured to set the second threshold value based on the standard deviation.

6. The nucleic acid sequence measuring apparatus (1) according to any one of claims 1 to 5,
wherein the image acquirer (12a) is configured to acquire images of the image acquisition region (BK) at different resolutions.

7. A nucleic acid sequence measuring method performed using a nucleic acid sequence measuring apparatus (1) which measures a target (TG) having a specific nucleic acid sequence included in a sample, the nucleic acid sequence measuring method comprising:
acquiring a difference between a first amount of light indicating an amount of fluorescent light emitted from a predefined measurement region (SP) of a nucleic acid sequence measuring device (DV) which emits fluorescence due to an addition of the target (TG) at a first time point before or immediately after an addition of the sample to the nucleic acid sequence measuring device (DV) and a second amount of light indicating an amount of fluorescent light emitted from the measurement region (SP) at a second time point after a predefined time has elapsed from the addition of the sample to the nucleic acid sequence measuring device (DV);
measuring the target (TG) based on the difference between the first amount of light and the second amount of light;
acquiring an image of an image acquisition region (BK) including at least the measurement region (SP); and
performing image processing on a first image that is an image of the image acquisition region (BK) acquired at the first time point and a second image that is an image of the image acquisition region (BK) acquired at the second time point to acquire the first amount of light and the second amount of light;
**characterized in that** the nucleic acid sequence measuring method comprises the steps of:
performing, as the image processing, a process of acquiring, as the first amount of light, an average gradation value of pixels forming an image of the measurement region (SP) included in the first image and a process of acquiring, as the second amount of light, an average gradation value of pixels forming an image of the measurement region (SP) included in the second image;
extracting a pixel in which a difference in gradation value for each of the pixels between the first image and the second image exceeds a predefined first threshold value, and
performing a process of acquiring, as the first amount of light, an average gradation value of the extracted pixels of the first image, and a process of acquiring, as the second amount of light, an average gradation value of the extracted pixels of the second image.

8. The nucleic acid sequence measuring method according to claim 7, further comprising:
acquiring a standard deviation of gradation values of pixels forming an image of the measurement region (SP) included in the first image; and
setting the first threshold value based on the standard deviation.

9. The nucleic acid sequence measuring method according to claim 7, further comprising:
performing, as the image processing, a process of acquiring a difference between gradation values of pixels forming an image of the measurement region (SP) included in the first image and gradation values of pixels forming an image of the measurement region (SP) included in the second image as a difference between the first amount of light and the second amount of light.

## Patentansprüche

1. Nukleinsäuresequenz-Messvorrichtung ("nucleic acid sequence measuring device", 1) zum Messen eines Ziels ("target", TG) mit einer spezifischen Nukleinsäuresequenz, das in einer Probe enthalten ist, die Nukleinsäuresequenz-Messvorrichtung (1) umfassend:
einen Detektor (12), der so konfiguriert ist, dass er Fluoreszenz erfasst, die von einer Nukleinsäuresequenz-Messvorrichtung (DV) emittiert wird, die aufgrund einer Zugabe des Ziels (TG) Fluoreszenz emittiert; und
einen Rechner (25), der so konfiguriert ist, dass er das Ziel (TG) basierend auf einer Differenz zwischen einer ersten Lichtmenge, die eine Menge an fluoreszierendem Licht angibt, das von einem vordefinierten Messbereich (SP) der Nukleinsäuresequenz-Messvorrichtung (DV) zu einem ersten Zeitpunkt vor oder unmittelbar nach einer Zugabe der Probe zu der Nukleinsäuresequenz-Messvorrichtung (DV) emittiert wird, und einer zweiten Lichtmenge, die eine Menge an fluoreszierendem Licht angibt, das von dem Messbereich (SP) an einem zweiten Zeitpunkt nachdem eine vordefinierten Zeit abgelaufen ist, nach der Zugabe der Probe zu der Nukleinsäuresequenz-Messvorrichtung (DV) emittiert, basierend auf einem Detektionsergebnis des Detektors (12), misst,
wobei der Detektor (12) eine Bildaufnahmevorrichtung ("image acquirer", 12a) umfasst, die so konfiguriert ist, dass sie ein Bild eines Bildaufnahmebereichs (BK) aufnimmt, der mindestens den Messbereich (SP) einschließt,
und
wobei der Rechner (25) konfiguriert ist, um Bildverarbeitung an einem ersten Bild, das ein Bild des Bildaufnahmebereichs (BK) ist, der zum ersten Zeitpunkt aufgenommen wurde, und einem zweiten Bild, das ein Bild des Bildaufnahmebereichs (BK) ist, der zum zweiten Zeitpunkt aufgenommen wurde, um die erste Lichtmenge und die zweite Lichtmenge zu erfassen,
**dadurch gekennzeichnet, dass** der Rechner (25) konfiguriert ist, um als die Bildverarbeitung, einen Prozess zum Erfassen, als erste Lichtmenge, eines durchschnittlichen Gradationswerts von Pixeln, die ein Bild von dem Messbereich (SP) bilden, der in dem ersten Bild enthalten ist, und einen Prozess zum Erfassen, als zweite Lichtmenge, eines durchschnittlichen Gradationswerts von Pixeln, eines durchschnittlichen Gradationswerts von Pixeln, die ein Bild von dem Messbereich (SP) bilden, der in dem zweiten Bild enthalten ist, durchzuführen,
dadurch, dass der Rechner (25) konfiguriert ist, um ein Pixel zu extrahieren, in dem eine Differenz im Gradationswert für jedes von den Pixeln zwischen dem ersten Bild und dem zweiten Bild einen vordefinierten ersten Schwellenwert überschreitet, und
dadurch, dass der Rechner (25) konfiguriert ist, um einen Prozess zum Erfassen, als die erste Lichtmenge, eines durchschnittlichen Gradationswerts von den extrahierten Pixeln des ersten Bildes und einen Prozess zum Erfassen, als die zweite Lichtmenge, eines durchschnittlichen Gradationswerts von den extrahierten Pixeln des zweiten Bildes, durchzuführen.

2. Die Nukleinsäuresequenz-Messvorrichtung (1) gemäß Anspruch 1,
wobei der Rechner (25) konfiguriert ist, um eine Standardabweichung von Gradationswerten von Pixeln zu erfassen, die ein Bild des Messbereichs (SP) bilden, der in dem ersten Bild enthalten ist, und konfiguriert ist, um den ersten Schwellenwert basierend auf der Standardabweichung festzulegen.

3. Die Nukleinsäuresequenz-Messvorrichtung (1) gemäß Anspruch 1,
wobei der Rechner (25) konfiguriert ist, um als die Bildverarbeitung, einen Prozess zum Erfassen einer Differenz zwischen Gradationswerten von Pixeln, die ein Bild des in dem ersten Bild enthaltenen Messbereichs (SP) bilden, und Gradationswerten von Pixeln, die ein Bild des Messbereichs (SP) bilden, der in dem zweiten Bild enthalten ist, als Differenz zwischen der ersten Lichtmenge und der zweiten Lichtmenge durchzuführen.

4. Die Nukleinsäuresequenz-Messvorrichtung (1) gemäß Anspruch 3,
wobei der Rechner (25) konfiguriert ist, um Pixel zu extrahieren, in denen die Differenz zwischen der ersten Lichtmenge und der zweiten Lichtmenge einen zweiten Schwellenwert überschreitet, und konfiguriert ist, um das Ziel (TG) basierend auf einer Anzahl der extrahierten Pixel zu messen.

5. Die Nukleinsäuresequenz-Messvorrichtung (1) gemäß Anspruch 4,
wobei der Rechner (25) konfiguriert ist, um eine Standardabweichung von Gradationswerten von Pixeln zu erfassen, die ein Bild eines Bereichs anderes als der in dem ersten Bild enthaltene Messbereich (SP) bilden, und konfiguriert ist, um den zweiten Schwellenwert basierend auf der Standardabweichung festzulegen.

6. Nukleinsäuresequenz-Messvorrichtung (1) gemäß irgendeinem der Ansprüche 1 bis 5,
wobei die Bildaufnahmevorrichtung (12a) konfiguriert ist, um Bilder des Bildaufnahmebereichs (BK) bei unterschiedlichen Auflösungen zu erfassen.

7. Verfahren zum Messen einer Nukleinsäuresequenz, durchgeführt unter Verwenden einer Nukleinsäuresequenz-Messvorrichtung (1), die ein Ziel (TG) mit einer spezifischen Nukleinsäuresequenz, das in einer Probe enthalten ist, misst, das Verfahren zum Messen einer Nukleinsäuresequenz umfassend:
Erfassen einer Differenz zwischen einer ersten Lichtmenge, die eine Menge an fluoreszierendem Licht angibt, das von einem vordefinierten Messbereich (SP) einer Nukleinsäuresequenz-Messvorrichtung (DV) emittiert, die aufgrund einer Zugabe des Ziels (TG) Fluoreszenz emittiert, zu einem ersten Zeitpunkt vor oder unmittelbar nach einer Zugabe der Probe zu der Nukleinsäuresequenz-Messvorrichtung (DV) und einer zweiten Lichtmenge, die eine Menge an fluoreszierendem Licht angibt, das von dem Messbereich (SP) zu einem zweiten Zeitpunkt nach Ablaufen einer vordefinierten Zeit nach der Zugabe der Probe zu der Nukleinsäuresequenz-Messvorrichtung (DV) emittiert;
Messen des Ziels (TG) basierend auf der Differenz zwischen der ersten Lichtmenge und der zweiten Lichtmenge; und
Erfassen eines Bildes von einem Bildaufnahmebereich (BK), der mindestens den Messbereich (SP) enthält; und Durchführen von Bildverarbeitung an einem ersten Bild, das ein Bild des Bildaufnahmebereichs (BK) ist, der zum ersten Zeitpunkt aufgenommen wurde, und einem zweiten Bild, das ein Bild des Bildaufnahmebereichs (BK) ist, der zum zweiten Zeitpunkt aufgenommen wurde, um die erste Lichtmenge und die zweite Lichtmenge zu erfassen; **dadurch gekennzeichnet, dass** das Verfahren zum Messen einer Nukleinsäuresequenz die Schritte umfasst: Durchführen, als die Bildverarbeitung, eines Prozesses zum Erfassen, als die erste Lichtmenge, eines durchschnittlichen Gradationswerts von Pixeln, die ein Bild von dem Messbereich (SP) bilden, der in dem ersten Bild enthalten ist, und eines Prozesses zum Erfassen, als zweite Lichtmenge, eines durchschnittlichen Gradationswerts von Pixeln, die ein Bild von dem Messbereich (SP) bilden, der in dem zweiten Bild enthalten ist; und
Extrahieren eines Pixels, in dem eine Differenz im Gradationswert für jedes von den Pixeln zwischen dem ersten Bild und dem zweiten Bild einen vordefinierten ersten Schwellenwert überschreitet, und
Durchführen eines Prozesses zum Erfassen, als die erste Lichtmenge, eines durchschnittlichen Gradationswerts von den extrahierten Pixeln des ersten Bildes, und eines Prozesses zum Erfassen, als die zweite Lichtmenge, eines durchschnittlichen Gradationswerts von den extrahierten Pixeln des zweiten Bildes.

8. Verfahren zum Messen einer Nukleinsäuresequenz gemäß Anspruch 7, ferner umfassend:
Erfassen einer Standardabweichung der Gradationswerte von Pixeln, die ein Bild von dem Messbereich (SP) bilden, der in dem ersten Bild enthalten ist; und
Festlegen des ersten Schwellenwerts basierend auf der Standardabweichung.

9. Verfahren zum Messen einer Nukleinsäuresequenz gemäß Anspruch 7, ferner umfassend:
Durchführen, als die Bildverarbeitung, eines Prozesses zum Erfassen einer Differenz zwischen Gradationswerten von Pixeln, die ein Bild von dem Messbereich (SP) bilden, der in dem ersten Bild enthalten ist, und
Gradationswerten von Pixeln, die ein Bild von dem Messbereich (SP) bilden, der in dem zweiten Bild enthalten ist, als eine Differenz zwischen der ersten Lichtmenge und der zweiten Lichtmenge.

## Revendications

1. Appareil de mesure de séquence d'acide nucléique (1) pour mesurer une cible (TG) présentant une séquence d'acide nucléique spécifique incluse dans un échantillon, l'appareil de mesure de séquence d'acide nucléique (1) comprenant :
un détecteur (12) configuré pour détecter une fluorescence émise par un dispositif de mesure de séquence d'acide nucléique (DV) qui émet une fluorescence en raison d'un ajout de la cible (TG) ; et
un calculateur (25) configuré pour mesurer la cible (TG) sur la base d'une différence entre une première quantité de lumière indiquant une quantité de lumière fluorescente émise par une région de mesure prédéfinie (SP) du dispositif de mesure de séquence d'acide nucléique (DV) à un premier instant avant ou immédiatement après un ajout de l'échantillon au dispositif de mesure de séquence d'acide nucléique (DV) et une deuxième quantité de lumière indiquant une quantité de lumière fluorescente émise par la région de mesure (SP) à un deuxième instant après l'écoulement d'un temps prédéfini depuis l'ajout de l'échantillon au dispositif de mesure de séquence d'acide nucléique (DV), sur la base d'un résultat de détection du détecteur (12),
dans lequel le détecteur (12) comprend un dispositif d'acquisition d'image (12a) configuré pour acquérir une image d'une région d'acquisition d'image (BK) incluant au moins la région de mesure (SP), et
dans lequel le calculateur (25) est configuré pour mettre en œuvre un traitement d'image sur une première image qui est une image de la région d'acquisition d'image (BK) acquise au premier instant et une deuxième image qui est une image de la région d'acquisition d'image (BK) acquise au deuxième instant pour acquérir la première quantité de lumière et la deuxième quantité de lumière,
**caractérisé en ce que**
le calculateur (25) est configuré pour mettre en œuvre, en tant que traitement d'image, un processus d'acquisition, en tant que première quantité de lumière, d'une valeur de gradation moyenne de pixels formant une image de la région de mesure (SP) incluse dans la première image et un processus d'acquisition, en tant que deuxième quantité de lumière, d'une valeur de gradation moyenne de pixels formant une image de la région de mesure (SP) incluse dans la deuxième image,
**en ce que**
le calculateur (25) est configuré pour extraire un pixel dans lequel une différence de valeur de gradation pour chacun des pixels entre la première image et la deuxième image dépasse une première valeur seuil prédéfinie, et
**en ce que**
le calculateur (25) est configuré pour mettre en œuvre un processus d'acquisition, en tant que première quantité de lumière, d'une valeur de gradation moyenne des pixels extraits de la première image, et un processus d'acquisition, en tant que deuxième quantité de lumière, d'une valeur de gradation moyenne des pixels extraits de la deuxième image.

2. Appareil de mesure de séquence d'acide nucléique (1) selon la revendication 1,
dans lequel le calculateur (25) est configuré pour acquérir un écart-type de valeurs de gradation de pixels formant une image de la région de mesure (SP) incluse dans la première image, et configuré pour régler la première valeur seuil sur la base de l'écart-type.

3. Appareil de mesure de séquence d'acide nucléique (1) selon la revendication 1,
dans lequel le calculateur (25) est configuré pour mettre en œuvre, en tant que traitement d'image, un processus d'acquisition d'une différence entre des valeurs de gradation de pixels formant une image de la région de mesure (SP) incluse dans la première image et des valeurs de gradation de pixels formant une image de la région de mesure (SP) incluse dans la deuxième image en tant que différence entre la première quantité de lumière et la deuxième quantité de lumière.

4. Appareil de mesure de séquence d'acide nucléique (1) selon la revendication 3,
dans lequel le calculateur (25) est configuré pour extraire des pixels dans lesquels la différence entre la première quantité de lumière et la deuxième quantité de lumière dépasse une deuxième valeur seuil, et configuré pour mesurer la cible (TG) sur la base d'un nombre des pixels extraits.

5. Appareil de mesure de séquence d'acide nucléique (1) selon la revendication 4,
dans lequel le calculateur (25) est configuré pour acquérir un écart-type de valeurs de gradation de pixels formant une image d'une région autre que la région de mesure (SP) incluse dans la première image, et configuré pour régler la deuxième valeur seuil sur la base de l'écart-type.

6. Appareil de mesure de séquence d'acide nucléique (1) selon l'une quelconque des revendications 1 à 5,
dans lequel le dispositif d'acquisition d'image (12a) est configuré pour acquérir des images de la région d'acquisition d'image (BK) à différentes résolutions.

7. Procédé de mesure de séquence d'acide nucléique mis en œuvre en utilisant un appareil de mesure de séquence d'acide nucléique (1) qui mesure une cible (TG) présentant une séquence d'acide nucléique spécifique incluse dans un échantillon, le procédé de mesure de séquence d'acide nucléique comprenant :
l'acquisition d'une différence entre une première quantité de lumière indiquant une quantité de lumière fluorescente émise par une région de mesure prédéfinie (SP) d'un dispositif de mesure de séquence d'acide nucléique (DV) qui émet une fluorescence en raison d'un ajout de la cible (TG) à un premier instant avant ou immédiatement après un ajout de l'échantillon au dispositif de mesure de séquence d'acide nucléique (DV) et une deuxième quantité de lumière indiquant une quantité de lumière fluorescente émise par la région de mesure (SP) à un deuxième instant après l'écoulement d'un temps prédéfini depuis l'ajout de l'échantillon au dispositif de mesure de séquence d'acide nucléique (DV) ;
la mesure de la cible (TG) sur la base de la différence entre la première quantité de lumière et la deuxième quantité de lumière ;
l'acquisition d'une image d'une région d'acquisition d'image (BK) incluant au moins la région de mesure (SP) ; et
la mise en œuvre d'un traitement d'image sur une première image qui est une image de la région d'acquisition d'image (BK) acquise au premier instant et une deuxième image qui est une image de la région d'acquisition d'image (BK) acquise au deuxième instant pour acquérir la première quantité de lumière et la deuxième quantité de lumière ;
**caractérisé en ce que** le procédé de mesure de séquence d'acide nucléique comprend les étapes de :
la mise en œuvre, en tant que traitement d'image, d'un processus d'acquisition, en tant que première quantité de lumière, d'une valeur de gradation moyenne de pixels formant une image de la région de mesure (SP) incluse dans la première image et d'un processus d'acquisition, en tant que deuxième quantité de lumière, d'une valeur de gradation moyenne de pixels formant une image de la région de mesure (SP) incluse dans la deuxième image ;
l'extraction d'un pixel dans lequel une différence de valeur de gradation pour chacun des pixels entre la première image et la deuxième image dépasse une première valeur seuil prédéfinie, et
la mise en œuvre d'un processus d'acquisition, en tant que première quantité de lumière, d'une valeur de gradation moyenne des pixels extraits de la première image, et d'un processus d'acquisition, en tant que deuxième quantité de lumière, d'une valeur de gradation moyenne des pixels extraits de la deuxième image.

8. Procédé de mesure de séquence d'acide nucléique selon la revendication 7, comprenant en outre :
l'acquisition d'un écart-type de valeurs de gradation de pixels formant une image de la région de mesure (SP) incluse dans la première image ; et
le réglage de la première valeur seuil sur la base de l'écart-type.

9. Procédé de mesure de séquence d'acide nucléique selon la revendication 7, comprenant en outre :
la mise en œuvre, en tant que traitement d'image, d'un processus d'acquisition d'une différence entre des valeurs de gradation de pixels formant une image de la région de mesure (SP) incluse dans la première image et des valeurs de gradation de pixels formant une image de la région de mesure (SP) incluse dans la deuxième image en tant que différence entre la première quantité de lumière et la deuxième quantité de lumière.
